# EUROPÄISCHE PATENTSCHRIFT

(11) **EP 1 770 395 B1**
(45) Veröffentlichungstag und Bekanntmachung des Hinweises auf die Patenterteilung: **10.11.2010**
(21) Anmeldenummer: 05021557.3
(22) Anmeldetag: 01.10.2005
(51) Int. Cl.: G01N 33/487

(54) **Analytisches System und Verfahren zu dessen Betrieb**
Analytical system and method of operation thereof
Système analytique et procédé pour son opération

(43) Veröffentlichungstag der Anmeldung: 04.04.2007
(73) Patentinhaber: F. Hoffmann-La Roche AG, 4070 Basel (CH); Roche Diagnostics GmbH, 68305 Mannheim (DE)
(72) Erfinder: Harttig, Herbert, Dr., 67434 Neustadt (DE)
(74) Vertreter: Pfiz, Thomas

(56) Entgegenhaltungen:
- EP-A- 1 360 935
- WO-A-03/082091
- WO-A-2005/040793
- US-A- 5 989 917

## Beschreibung

Die Erfindung betrifft ein analytisches System insbesondere zur Durchführung von Patientenselbstkontrollen wie Blutzuckertests. Die Erfindung betrifft weiter ein Verfahren zum Betrieb eines solchen Systems.

Derartige Systeme dienen vor allem Diabetikern zur täglich mehrfach durchgeführten Blutzucker-Selbstkontrolle im Rahmen einer Insulinbehandlung. Damit auch von Laien die erforderlichen Schritte einfach und schnell vorgenommen werden können, ist es wünschenswert, einen weitgehend automatischen Messablauf in einem kompakten Handgerät zu realisieren. Dabei soll dem Benutzer eine einfachere Handhabung und größere Flexibilität ermöglicht werden, was die Bereitstellung von Testelementen in messgerätegängigen Magazinen beinhaltet. Die Testverarbeitung erfolgt generell mit einer chemischen Nachweisreaktion, deren Fortschritt bzw. Endpunkt optisch oder elektrochemisch erfasst wird. Die Korrelation zwischen erhaltenem Messsignal und Analytkonzentration unterliegt dabei gewissen Schwankungen, die sich von Fertigungslot zu Fertigungslot unterscheiden können. Um diese Schwankungen zu berücksichtigen, wurden bereits magazinseitige Codes eingeführt, mit deren Hilfe das Messsignal geräteseitig so korrigiert werden kann, dass der ausgegebene Wert eine bessere Übereinstimmung mit der tatsächlichen Analytkonzentration aufweist als das Rohsignal.

Die WO 2005/040793 A offenbart ein gattungsgemäßes System, wobei die Messeinrichtung mit einem gesonderten Lese/Schreibspeicher kommuniziert, um anhand einer eindeutigen Kennung eine Kalibrierung und auch eine Zählerrevision für ein gegebenes Magazin vorzunehmen. Ein ähnliches System ist auch der WO 03/082091 A bekannt, wobei auch dort ein Testzähler nicht explizit erwähnt ist und ein rückschreibbares Speichermodul vorgesehen ist, das bevorzugt mit dem Magazin verbunden ist.

Aus der WO2005/065828 ist ein Analysehandgerät zum Aufnehmen eines auswechselbaren Magazins bekannt. Das Magazin kann dort als Trommelmagazin mehrere in Umfangsrichtung verteilte Kammern zur Aufnahme jeweils eines Teststreifens aufweisen, wobei die Stirnseiten des Magazins mit Siegelfolie verschlossen sein können und bei einer Streifenentnahme die Siegelfolie durchbrochen wird. Insbesondere nach einer vorübergehenden Entnahme eines Trommelmagazins kann eine erfolglose Betätigung der Entnahmeeinrichtung dadurch vermieden werden, dass eine Prüfeinrichtung ein Signal erzeugt, wenn die in der Entnahmeposition befindliche Kammer nicht mit Siegelfolie verschlossen ist.

Ausgehend hiervon liegt der Erfindung die Aufgabe zugrunde, die im Stand der Technik bekannten Systeme weiter zu verbessern und mit einfachen Mitteln eine erhöhte Verbraucherfreundlichkeit und Verfahrenssicherheit zu gewährleisten.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine geräteseitige Verbrauchserfassung der Testeinheiten zu ermöglichen. Für den wahlweisen Einsatz mehrer Magazine wird vorgeschlagen, dass der Code eine eindeutige Magazinkennung für das Magazin umfasst, dass die Messeinrichtung einen die Verarbeitung einer Testeinheit erfassenden magazinunabhängigen Testzähler und einen Zählerspeicher zum Speichern der Magazinkennung und eines zugeordneten Zählerstandes des Testzählers aufweist, und dass der Zählerspeicher eine Vielzahl von Speicherplätzen für die Speicherung einer Vielzahl von Magazinkennungen und zugeordneten Zählerständen aufweist. Dadurch kann auch auf ein Rückschreiben der Verbrauchsinformation auf das Magazin verzichtet werden, so dass dieses als Austauschartikel weiter vereinfacht werden kann. Der Rückschreibeverzicht erlaubt auch eine günstigere Auslegung der Codeleseeinrichtung. Da in der Geräteelektronik die erforderlichen Bausteine beispielsweise in Form programmierbarer Mikroprozessoren ohnehin für die Testverarbeitung vorhanden sind, ist auch auf dieser Seite keine aufwändige zusätzliche Implementierung erforderlich.

Vorteilhafterweise enthält der Testzähler eine Arithmetikeinheit beispielsweise in Form einer Programmroutine auf einem Mikroprozessor, welche den zu der erfassten Magazinkennung eines Magazins gespeicherten Zählerstand entsprechend dem Verbrauch von Testeinheiten aktualisiert, wobei der Zählerstand die Anzahl der verbrauchten oder noch verfügbaren Testeinheiten eines betreffenden Magazins angibt.

Um den Gebrauchskomfort zu erhöhen, ist es von Vorteil, wenn der Zählerstand für das eingesetzte Magazin auf einer Anzeige für einen Benutzer anzeigbar ist.

Hierbei wird vorgeschlagen, dass der Testzähler einen Vergleicher zum Vergleich der eingelesenen Magazinkennung mit in dem Zählerspeicher vorhandenen Magazinkennungen aufweist. Abhängig von diesem Vergleich ist es vorgesehen, dass der Testzähler einer noch nicht gespeicherten Magazinkennung eines eingewechselten Magazins einen Anfangszählerstand zuordnet, und dass der Testzähler bei Übereinstimmung der Magazinkennung eines eingewechselten Magazins mit einer gespeicherten Magazinkennung den im Zählerspeicher zugeordneten Zählerstand ausliest und weiterzählt.

Vorteilhafterweise umfasst die Messeinrichtung einen optisch, magnetisch, elektrisch oder elektromagnetisch arbeitenden Codeleser zur Erfassung mindestens der Magazinkennung. Dabei kann der Code als Barcode, insbesondere 2-DBarcode, Magnetstreifen, elektronischer Speicherbaustein, insbesondere EPROM, oder Transponder an dem Magazin angebracht sein. Für die Erfassung des Verbrauchszustands sollte der Code auch die Gesamtzahl der Testeinheiten eines Magazins enthalten.

Besonders vorteilhaft ist der Einsatz in einem Handgerät zum Einwechseln eines Magazins als Verbrauchseinheit.

Die im Magazin bevorrateten Testeinheiten sind bevorzugt als Testband oder Teststreifen zur Beaufschlagung mit Körperflüssigkeit ausgebildet.

In verfahrensmäßiger Hinsicht wird die eingangs genannte Aufgabe dadurch gelöst, dass in einer Vielzahl von Speicherplätzen des Zählerspeichers eine Vielzahl von Magazinkennungen und zugeordneten Zählerständen gespeichert wird.

Im Folgenden wird die Erfindung anhand eines in der Zeichnung schematisch dargestellten Ausführungsbeispiels näher erläutert. Es zeigen
- Fig. 1: ein Blockschaltbild eines analytischen Systems für Blutzuckertests mit Testzähler; und
- Fig. 2: das System als Handgerät in einer perspektivischen Darstellung.

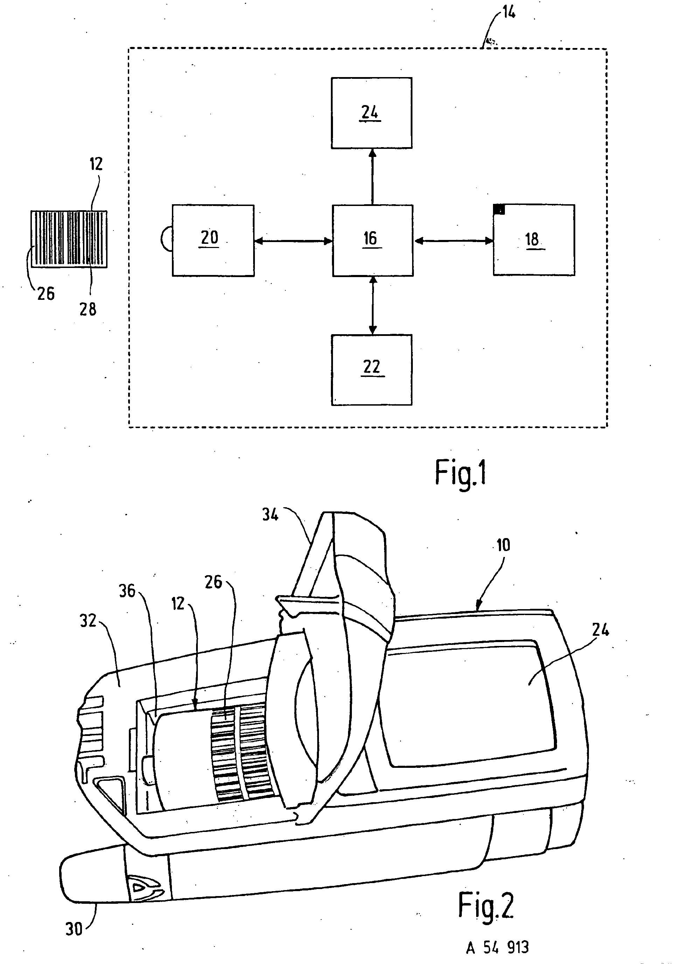

Das in Fig. 1 gezeigte Blutzucker-Testsystem ermöglicht Patientenselbstkontrollen mittels Handgerät 10 und darin einsetzbarem Magazin 12 zur Bereitstellung einer Vielzahl von auf einen Analyten (Blutglucose) ansprechenden Testeinheiten. Die Testeinheiten können beispielsweise durch Teststreifen in einem Streifenmagazin oder durch Bandabschnitte in einem Bandmagazin (Kassette) gebildet sein und lassen sich sukzessive in einer geräteseitigen Messeinrichtung 14 verarbeiten, um dem Benutzer in sog. Spot-Messungen ein Momentanbild seines Blutzuckerspiegels zu liefern.

Wie in Fig. 1 veranschaulicht, umfasst die Messeinrichtung 14 einen Testzähler 16, einen Zählerspeicher 18, einen Codeleser 20, einen Testprozessor 22 sowie eine Anzeige 24. Das Magazin 12 ist mit einem durch den Codeleser 20 abtastbaren Strichcode 26 versehen, welcher eine eindeutige Magazinkennung 28 für das momentan eingesetzte Magazin 12 umfasst. Dieses lässt sich als Verbrauchseinheit durch den Benutzer auswechseln. Dabei ist es auch denkbar, dass ein angebrochenes Magazin mit einem Restbestand an unverbrauchten Testeinheiten erneut eingewechselt wird. Auch in diesem Fall soll der Benutzer durch das System auf einfache Weise über die Zahl der verbrauchten oder noch verfügbaren Testeinheiten informiert werden.

Fig. 2 zeigt eine mögliche Ausführung in Form eines kombinierten Geräts, welches eine Stechhilfe 30 zum Gewinnen einer kleinen Blutmenge aus einem Körperteil und ein Analyseteil 32 für die Untersuchung der Blutprobe umfasst. Der Analyseteil 32 weist einen mittels Deckel 34 verschließbaren Magazinschacht 36 auf, in welchem ein Trommelmagazin 12 mit einer Mehrzahl von nicht gezeigten Teststreifen als Testeinheiten einsetzbar ist. Ein geeignetes Trommelmagazin und eine Entnahmevorrichtung für Teststreifen in einem Analysehandgerät sind beispielsweise aus der WO2005/065828 A1 bekannt. Aus dem Trommelmagazin lassen sich die Teststreifen einzeln zur Beaufschlagung mit Blut ausschieben, wobei der Nachweis des Analyten in an sich bekannter Weise elektrochemisch oder photometrisch erfolgt. Anschließend kann durch Rotation des Trommelmagazins 26 ein neuer Teststreifen für eine Folgemessung aktiviert werden. Ein solches Trommelmagazin 26 kann beispielsweise 17 Teststreifen enthalten, während bei Einsatz eines Bandmagazins in einem dafür ausgelegten Gerät eine erheblich höhere Gesamtzahl an Testeinheiten realisierbar ist. Ein Bandmagazin als Kassette in einem Körperflüssigkeits-Testgerät geht beispielsweise aus der EP-A 1 424 040 hervor.

Für die Verbrauchserfassung ist der magazinunabhängig arbeitende Testzähler 16 vorgesehen, welcher unter Rückschreibeverzicht ohne magazinseitigen Eingriff in Kombination mit dem Zählerspeicher 18 eine einfache geräteseitige Hinterlegung des Verbrauchszustandes eines oder mehrerer Magazine 12 ermöglicht. Zu diesem Zweck weist der Testzähler eine zweckmäßig softwaremäßig realisierte Arithmetikeinheit auf, welche den zu der erfassten Magazinkennung 28 eines Magazins 12 gespeicherten Zählerstand entsprechend dem Verbrauch von Testeinheiten aktualisiert. Hierbei kann der Zählerstand die Anzahl der verbrauchten oder noch verfügbaren Testeinheiten des betreffenden eingesetzten Magazins angeben und auf der Anzeige 24 für den Benutzer wiedergegeben werden.

Im Folgenden wird die Verfahrensweise zur Ermittlung und Speicherung des Zählerstandes näher erläutert. Der Code 26 enthält neben der Magazinkennung 28 weitere Informationen über die Gesamtzahl der Tests pro Magazin, über Herstellungscharge, Herstellungsdatum, Haltbarkeit und ggf. über Parameter zur Berechnung des korrekten Zusammenhangs zwischen dem Messsignal und der Analytkonzentration in der Probe. Der Code 26 kann als Strichcode mittels Laser auf ein geeignetes Etikett geschrieben und auf dem Magazin 12 fixiert werden. Denkbar ist auch der Einsatz von Codeträgern mit höherer Informationsdichte, beispielsweise Magnetstreifen, elektronische Speicherbausteine (z. B. EPROM) oder Transponder an dem Magazin 12, wobei in jedem Fall auf ein Rückschreiben des Zählerstandes verzichtet wird, um eine möglichst einfache Gerätekonstruktion und die Verwendung kostengünstiger Datencodes zu erlauben.

Beim Einwechseln eines Magazins 12 wird die Magazinkennung 28 mittels des Codelesers 20 berührungslos optisch eingelesen und durch einen Vergleicher des Testzählers 16 mit in dem Zählerspeicher 18 vorhandenen Magazinkennungen verglichen. Bei einer noch nicht gespeicherten Magazinkennung wird durch den Testzähler ein Anfangszählerstand zugeordnet, während bei Übereinstimmung mit einer gespeicherten Magazinkennung der im Zählerspeicher 18 zugeordnete Zählerstand ausgelesen wird. Der Zählerspeicher 18 ist durch eine Vielzahl von Speicherplätzen dafür ausgelegt, eine Vielzahl von Magazinkennungen 28 und zugeordneten Zählerständen zu hinterlegen.

Der Testprozessor 22 umfasst alle gerätetechnischen Einheiten für den automatischen Prozessablauf bei der Probenuntersuchung. Die Verarbeitung kann dabei neben der regulären analytischen Prozessierung einer Testeinheit auch deren Verwerfung umfassen, wenn beispielsweise die Testeinheit verfallen oder der betreffende Test missglückt ist. Bei der Verarbeitung einer neuen Testeinheit durch den Testprozessor 22 wird der Testzähler 16 um eine Zähleinheit weitergeschaltet. Der im Testzähler 16 entsprechend weitergezählte Zählerstand wird jeweils in Zuordnung zu der Magazinkennung 28 auf den Zählerspeicher 18 zurückgeschrieben, so dass auch beim Ausschalten des Gerätes oder Auswechseln des Magazins die aktuelle Datenkombination erhalten bleibt. Wird ausgehend von der bekannten Gesamtzahl der Tests beim Herunterzählen der Zählerstand "Null" erreicht, so kann der Datenbereich des nunmehr verbrauchten Magazins im Zählerspeicher 18 gelöscht werden. Es ist aber auch möglich, alle Datenkombinationen im Zählerspeicher zu belassen. Damit können retrospektiv alle individuellen Daten ausgelesen werden, die den jemals im Gerät eingesetzten Magazinen zugeordnet sind.

Der Transfer von angebrauchten Magazinen bzw. Kassetten zwischen verschiedenen Messgeräten ist nicht vorgesehen. Bei einem solchen Transfer könnte unter Umständen eine gebrauchte Testeinheit im zweiten Gerät nochmals zur Messung gelangen, sofern eine Wiederverwendung physikalisch überhaupt möglich ist. Dies kann jedoch durch eine einfache Kontrollmessung, z.B. von photometrischen oder elektrischen Leerwerten ohne zusätzlichen Geräteaufwand erkannt und eine Fehlmessung somit unterbunden werden. Dadurch wird ein Benutzer auch bei krasser Fehlbedienung zuverlässig vor falschen Messwerten und daraus abgeleiteter falscher Behandlung geschützt.

## Patentansprüche

1. Analytisches System insbesondere zur Durchführung von Patientenselbstkontrollen wie Blutzuckertests, mit einem wechselbaren Magazin (12) zur Bereitstellung einer Vielzahl von auf einen Analyten ansprechenden Testeinheiten und einer Messeinrichtung (14) zur Verarbeitung der Testeinheiten, wobei das Magazin (12) mit einem durch die Messeinrichtung (14) erfassbaren Code (26) versehen ist und der Code (26) eine eindeutige Magazinkennung (28) für das Magazin (12) umfasst, wobei die Messeinrichtung (14) einen die Verarbeitung einer Testeinheit erfassenden magazinunabhängigen Testzähler (16) und einen Zählerspeicher (18) zum Speichern der Magazinkennung (28) und eines zugeordneten Zählerstandes des Testzählers (16) aufweist, und wobei der Zählerspeicher (18) eine Vielzahl von Speicherplätzen für die Speicherung einer Vielzahl von Magazinkennungen (28) und zugeordneten Zählerständen aufweist.

2. Analytisches System nach Anspruch 1, **dadurch gekennzeichnet, dass** der Testzähler (16) eine Arithmetikeinheit aufweist, welche den zu der erfassten Magazinkennung (28) eines Magazins (12) gespeicherten Zählerstand entsprechend dem Verbrauch von Testeinheiten aktualisiert.

3. Analytisches System nach Anspruch 1 oder 2, **dadurch gekennzeichnet, dass** der Zählerstand die Anzahl der verbrauchten oder noch verfügbaren Testeinheiten eines betreffenden Magazins (12) angibt.

4. Analytisches System nach einem der Ansprüche 1 bis 3, **dadurch gekennzeichnet, dass** der Zählerstand für das eingesetzte Magazin (12) auf einer Anzeige (24) für einen Benutzer anzeigbar ist.

5. Analytisches System nach einem der Ansprüche 1 bis 4, **dadurch gekennzeichnet, dass** der Testzähler (16) einen Vergleicher zum Vergleich der eingelesenen Magazinkennung (28) mit in dem Zählerspeicher (18) vorhandenen Magazinkennungen aufweist.

6. Analytisches System nach einem der Ansprüche 1 bis 5, **dadurch gekennzeichnet, dass** der Testzähler (16) einer noch nicht gespeicherten Magazinkennung (28) eines eingewechselten Magazins (12) einen Anfangszählerstand zuordnet.

7. Analytisches System nach einem der Ansprüche 1 bis 6, **dadurch gekennzeichnet, dass** der Testzähler (16) bei Übereinstimmung der Magazinkennung (28) eines eingewechselten Magazins (12) mit einer gespeicherten Magazinkennung (28) den im Zählerspeicher (18) zugeordneten Zählerstand ausliest und weiterzählt.

8. Analytisches System nach einem der Ansprüche 1 bis 7, **dadurch gekennzeichnet, dass** die Messeinrichtung (14) einen optisch, magnetisch, elektrisch oder elektromagnetisch arbeitenden Codeleser (20) zur Erfassung mindestens der Magazinkennung (28) aufweist.

9. Analytisches System nach einem der Ansprüche 1 bis 8, **dadurch gekennzeichnet, dass** der Code (26) als Barcode, insbesondere 2-DBarcode, Magnetstreifen, elektronischer Speicherbaustein, insbesondere EPROM, oder Transponder an dem Magazin (12) angebracht ist.

10. Analytisches System nach einem der Ansprüche 1 bis 9, **dadurch gekennzeichnet, dass** der Code (26) die Gesamtzahl der Testeinheiten eines Magazins (12) enthält.

11. Analytisches System nach einem der Ansprüche 1 bis 10, **dadurch gekennzeichnet, dass** die Testeinheiten als Testband oder Teststreifen zur Beaufschlagung mit Körperflüssigkeit ausgebildet sind.

12. Verfahren zum Betrieb eines analytischen Systems insbesondere zur Durchführung von Patientenselbstkontrollen wie Blutzuckertests, bei dem durch ein wechselbares Magazin (12) eine Vielzahl von auf einen Analyten ansprechenden Testeinheiten bereitgestellt werden und die Testeinheiten in einer Messeinrichtung (14) verarbeitet werden, wobei eine eindeutige Magazinkennung (28) des Magazins (12) erfasst wird, der Magazinkennung (28) in einem Zählerspeicher (18) ein Zählerstand für die Anzahl der verbrauchten oder verfügbaren Testeinheiten des Magazins (12) zugeordnet wird und bei Verarbeitung einer Testeinheit der Zählerstand ohne magazinseitigen Eingriff entsprechend weitergezählt wird, **dadurch gekennzeichnet, dass** in einer Vielzahl von Speicherplätzen des Zählerspeichers (18) eine Vielzahl von Magazinkennungen (28) und zugeordneten Zählerständen gespeichert wird.

## Claims

1. Analytical system especially for carrying out patient self-monitoring such as blood sugar tests comprising a replaceable magazine (12) for providing a plurality of test units that react to an analyte and a measuring device (14) for processing the test units, wherein the magazine (12) is provided with a code (26) that can be registered by the measuring device (14) and the code (26) comprises an unique magazine identifier (28) for the magazine (12), wherein the measuring device (14) has a magazine-independent test counter (16) which registers the processing of a test unit and a counter memory (18) to store the magazine identifier (28) and a corresponding count of the test counter (16), and wherein the counter memory (18) has a plurality of storage locations for the storage of a plurality of magazine identifiers (28) and associated counts.

2. Analytical system according to claim 1, **characterized in that** the test counter (16) contains an arithmetic unit which updates the count stored for the registered magazine identifier (28) of a magazine (12) according to the consumption of test units.

3. Analytical system according to claim 1 or 2, **characterized in that** the count gives the number of test units of a respective magazine (12) that are used or are still available.

4. Analytical system according to one of the claims 1 to 3, **characterized in that** the count for the magazine in use (12) can be displayed for the user on a display (24).

5. Analytical system according to one of the claims 1 to 4, **characterized in that** the test counter (16) has a comparator to compare the magazine identifier (28) that is read in with the magazine identifiers that are present in the counter memory (18).

6. Analytical system according to one of the claims 1 to 5, **characterized in that** the test counter (16) allocates an initial count to a magazine identifier (28) of a newly inserted magazine (12) that has not yet been stored.

7. Analytical system according to one of the claims 1 to 6, **characterized in that** when the magazine identifier (28) of a newly inserted magazine (12) agrees with a stored magazine identifier (28), the test counter (16) reads out the corresponding count in the counter memory (18) and continues to count.

8. Analytical system according to one of the claims 1 to 7, **characterized in that** the measuring device (14) comprises an optical, magnetic, electric or electromagnetic code reader (20) to register at least the magazine identifier (28).

9. Analytical system according to one of the claims 1 to 8, **characterized in that** the code (26) is attached to the magazine (12) as a bar-code, in particular a 2-D bar-code, magnetic strip, electronic memory component, in particular an EPROM or transponder.

10. Analytical system according to one of the claims 1 to 9, **characterized in that** the code (26) includes the total count of test units of a magazine (12).

11. Analytical system according to one of the claims 1 to 10, **characterized in that** the test units are in the form of a test tape or test strip to which body fluid can be applied.

12. Method for operating an analytical system, especially for carrying out patient self-monitoring such as blood sugar tests in which a plurality of test units that react to an analyte are provided by a replaceable magazine (12) and the test units are processed in a measuring device (14), wherein an unique magazine identifier (28) of the magazine (12) is registered, the magazine identifier (28) is allocated a count for the number of used or available test units of the magazine (12) in a counter memory (18) and when a test unit has been processed, the counter reading is advanced without intervention on the magazine, **characterized in that** a plurality of magazine identifiers (28) and associated counts are stored in a plurality of storage locations of the counter memory (18).

## Revendications

1. Système analytique, en particulier pour la mise en oeuvre d'autocontrôles réalisés par des patients, tels que des tests de glycémie, comprenant un magasin (12) qui peut être changé pour la mise à disposition d'une pluralité d'unités de test sensibles à un analyte et un dispositif de mesure (14) pour le traitement des unités de test, le magasin (12) étant muni d'un code (26) qui peut être enregistré par le mécanisme de mesure (14) et le code (26) comprenant un indicatif de magasin univoque (28) pour le magasin (12), dans lequel le dispositif de mesure (14) présente un compteur de test (16) indépendant du magasin enregistrant le traitement d'une unité de test et une mémoire de compteur (18) pour mémorisation de l'indicatif de magasin (28) et d'un état de compteur correspondant du compteur de test (16), et dans lequel la mémoire de compteur (18) présente une pluralité d'endroits de mémoire pour la mémorisation d'une pluralité d'indicatifs de magasins (28) et d'états de compteurs correspondants.

2. Système analytique selon la revendication 1, **caractérisé en ce que** le compteur de test (16) présente une unité arithmétique qui actualise l'état de compteur enregistré pour l'indicatif de magasin enregistré (28) d'un magasin (12) de manière correspondante à la consommation des unités de test.

3. Système analytique selon la revendication 1 ou 2, **caractérisé en ce que** l'état de compteur indique le nombre des unités de test consommées ou encore disponibles d'un magasin correspondant (12).

4. Système analytique selon l'une quelconque des revendications 1 à 3, **caractérisé en ce que** l'état de compteur pour le magasin mis en oeuvre (12) peut être indiqué sur un affichage (24) pour un utilisateur.

5. Système analytique selon l'une quelconque des revendications 1 à 4, **caractérisé en ce que** le compteur de test (16) présente un comparateur pour comparer l'indicatif de magasin entré par lecture (28) aux indicatifs de magasins présents dans la mémoire de compteur (18).

6. Système analytique selon l'une quelconque des revendications 1 à 5, **caractérisé en ce que** le compteur de test (16) d'un indicatif de magasin (28) d'un magasin qui a été changé (12) et qui n'a pas encore été enregistré attribue un état de compteur de départ.

7. Système analytique selon l'une quelconque des revendications 1 à 6, **caractérisé en ce que** le compteur de test (16), lors d'une concordance de l'indicatif de magasin (28) d'un magasin qui a été changé (12) avec un indicatif de magasin enregistré (28) lit l'état de compteur attribué dans la mémoire de compteur (18) et poursuit son comptage.

8. Système analytique selon l'une quelconque des revendications 1 à 7, **caractérisé en ce que** le dispositif de mesure (14) présente un lecteur de code (20) travaillant de manière optique, de manière magnétique, de manière électrique ou de manière électromagnétique pour l'enregistrement d'au moins l'indicatif de magasin (28).

9. Système analytique selon l'une quelconque des revendications 1 à 8, **caractérisé en ce que** le code (26) est appliqué au magasin (12) sous la forme d'un code barre, en particulier d'un code barre en deux dimensions, sous la forme de bandes magnétiques, sous la forme d'un élément d'enregistrement électronique, en particulier d'une EPROM, ou sous la forme d'un transpondeur.

10. Système analytique selon l'une quelconque des revendications 1 à 9, **caractérisé en ce que** le code (26) renferme le nombre total des unités de test d'un magasin (12).

11. Système analytique selon l'une quelconque des revendications 1 à 10, **caractérisé en ce que** les unités de test sont réalisées sous la forme d'une bande test ou d'une bandelette réactive destinée à une sollicitation avec un fluide organique.

12. Procédé pour la mise en service d'un système analytique, en particulier pour la mise en oeuvre d'autocontrôles réalisés par des patients tels que des tests de glycémie, dans lequel, via un magasin (12) qui peut être changé, on présente une pluralité d'unités de test sensibles à un analyte et on traite les unités de test d'un dispositif de mesure (14), un indicatif de magasin univoque (28) du magasin (12) étant enregistré, un état de compteur pour le nombre des unités de test consommées ou disponibles du magasin (12) étant attribué à l'indicatif de magasin (28) dans une mémoire de compteur (18) et, lors du traitement d'une unité de test, l'état de compteur étant soumis à un comptage ultérieur en l'absence d'intervention côté magasin, **caractérisé en ce que**, dans une pluralité d'endroits de mémoire de la mémoire de compteur (18), on mémorise une pluralité d'indicatifs de magasins (28) et d'états de compteurs correspondants.
